# FASCICULE DE BREVET EUROPEEN

(11) **EP 0 699 428 B1**
(45) Date de publication et mention de la délivrance du brevet: **30.07.1997**
(21) Numéro de dépôt: 95401842.0
(22) Date de dépôt: 04.08.1995
(51) Int. Cl.: A61K 7/00, A61K 7/48, A61K 9/127, A61K 35/78

(54) **Composition cosmétique ou dermatologique contenant des extraits de farine de mais encapsulés**
Kosmetische oder dermatologische Zusammensetzung, die eingekapselte Maismehlextrakte enthält
Cosmetic or dermatological composition comprising encapsulated extracts of cornflour

(30) Priorité: 31.08.1994 FR 9410488
(43) Date de publication de la demande: 06.03.1996
(73) Titulaire: L'OREAL, 75008 Paris (FR)
(72) Inventeur: Koulbanis, Constantin, F-94270 Le Kremlin-Bicetre (FR)
(74) Mandataire: Lhoste, Catherine

(56) Documents cités:
- EP-A- 0 469 232
- WO-A-83/00433
- FR-A- 2 315 991
- MANUF. CHEM., Juin 1993 pages 31 - 33 P. ALEXANDER 'Cosmetic raw materials.'

## Description

L'invention se rapporte à une composition cosmétique et/ou dermatologique contenant des extraits de farine de maïs encapsulés. Cette composition peut être blanche ou colorée, et se présenter notamment sous de forme d'émulsion eau-dans-huile ou huile-dans-eau, de pâte, de gel lipophile ou hydrophile, de solution liquide lipophile ou hydrophile.

Cette composition peut être utilisée pour le soin de la peau, aussi bien du visage que du corps humain y compris le cuir chevelu et les muqueuses, le soin des cheveux et aussi pour le traitement thérapeutique de la peau et des muqueuses. Ce traitement dépend certes de l'extrait mais aussi des actifs qui lui sont éventuellement associés. En particulier la composition est destinée à la régénération des cellules et des tissus de la peau et/ou l'activation de leur développement. Ainsi, la composition peut être utilisée pour prévenir et/ou lutter contre le vieillissement cutané ainsi que pour la cicatrisation des plaies.

En outre, la composition de l'invention peut être une crème de soin, blanche ou colorée, un baume, un onguent dermatologique, une lotion capillaire un lait corporel ou un sérum.

Il est connu depuis longtemps d'utiliser des protéines ou des hydrolysats dans des compositions cosmétiques en vue de lutter contre le vieillissement cutané. A cet effet, on peut se référer au document EP-A-457 565 enseignant l'emploi de protéine de lait pour activer la prolifération de cellules cutanées humaines et au document WO 84/03835 enseignant l'emploi de germes de blé ou de soja pour régénérer les cellules et les tissus cutanés. Malheureusement, ces compositions connues présentent une efficacité insuffisante. Or, les êtres humains et plus spécialement les femmes sont de plus en plus exigeants quant aux résultats obtenus pour les produits de soin et notamment en ce qui concerne les produits destinés à lutter contre les signes de vieillissement.
Par ailleurs, il est connu de l'article 〈〈 Cosmetic raw materials 〉〉, Manuf. Chem., Juin 1993, p.31-33 d'utiliser un extrait de blé lyophilisé comme régulateur des fonctions de la peau.

Les signes caractéristiques du vieillissement se traduisent par une modification de la structure et de la fonction cutanées. Ces signes sont notamment les rides profondes, en augmentation avec l'âge et les ridules. On constate en outre une désorganisation du "grain" de la peau, conférant au micro-relief de la peau un aspect moins régulier et anisotrope. En outre, le teint de la peau apparaît plus pâle et plus jaune. Un autre signe clinique du vieillissement est l'aspect sec et rêche de la peau. Enfin, la peau perd en fermeté et en tonicité.

On constate donc que les signes cliniques de vieillissement cutané résultent essentiellement d'un dysfonctionnement des principaux mécanismes biologiques intervenant au niveau de la peau.

Il subsiste donc le besoin d'une composition cosmétique et/ou dermatologique destinée notamment au traitement du vieillissement présentant une efficacité améliorée par rapport aux compositions connues.

L'invention a justement pour objet une composition cosmétique et/ou dermatologique contenant, dans un milieu cosmétiquement et/ou dermatologiquement acceptable, au moins un extrait de farine de maïs encapsulé dans des vésicules lipidiques.

Ces vésicules sont formées d'une membrane constituée par un ou plusieurs feuillets concentriques comportant chacun une ou plusieurs couches bimoléculaires de lipides amphiphiles, ces lipides étant soit ioniques, soit non-ioniques. Lorsque les lipides des membranes sont tous ioniques, les vésicules sont généralement connues sous le nom de liposomes.

Les vésicules utilisables dans l'invention sont en particulier celles décrites dans les documents FR-A-2 315 991, FR-A-2 485 921, FR-A-2 490 504.

A titre d'exemple, les lipides ioniques utilisables dans l'invention sont des phospholipides naturels éventuellement hydrogénés, et notamment la lécithine de plantes ou d'oeuf ou la sphingomyéline, les phospholipides synthétiques saturés tels que la dipalmitoylphosphatidylcholine ou la lécithine hydrogénée, les composés cationiques ou quaternaires comme le chlorure ou le bromure de didocétyl- ou distéaryl-diméthylammonium.

A titre d'exemple, les lipides non-ioniques sont :
- les éthers de polyglycérol linéaires ou ramifiés de formules respectives :

   R-(OCH₂-CHOH-CH₂)ₙ-OH et R-(O-CH₂-CH)ₙ-OH

   CH₂-OH

   où n est un entier allant de 1 à 6 et R est une chaîne aliphatique linéaire ou ramifiée, saturée ou insaturée de 12 à 30 atomes de carbone, les radicaux hydrocarbonés des alcools de lanoline ou les restes hydroxy-2 alkyle des α-diols à longue chaîne ;
- les alcools gras polyoxyéthylénés ;
- les esters de polyols oxyéthylénés ou non et, en particulier les esters de sorbitol polyoxyéthylénés ;
- les glycolipides d'origine naturelle ou synthétique comme les cérébrosides.

En vue de modifier la perméabilité et/ou la charge superficielle des vésicules lipidiques, on peut utiliser divers additifs tels que les alcools, diols et triols à longue chaîne comme le phytanetriol, les stérols comme le cholestérol ou le phytostérol polyoxyéthyléné, les amines à longue chaîne ou leurs dérivés ammonium-quaternaires, les dihydroxyalkylamines dont le radical alkyle a 6 à 16 atomes de carbone et/ou un additif ionique comme les esters phosphoriques d'alcools gras ou leurs sels alcalins tels que le dicétylphosphate, le dicétylphosphate de sodium, les alkylsulfates tels que le cétylsulfate de sodium, les sels alcalins du cholestérol sulfate ou du cholestérol phosphate, les lipoaminoacides et leurs sels tels que les acylglutamates de sodium, le sel de sodium de l'acide phosphatidique.

Les compositions de l'invention peuvent se présenter sous forme d'émulsion huile-dans-eau, eau-dans-huile, de gel aqueux ou huileux, de solution lipophile ou hydrophile. Elles peuvent avoir l'aspect d'une crème, d'un sérum, d'une lotion, d'une pommade ou onguent.

Les huiles et/ou les corps gras, les gélifiants, les agents de mise en suspension, et les tensioactifs nécessaires à la formulation et stabilisation de ces compositions sont ceux classiquement utilisés dans le domaine cosmétique et/ou dermatologique. Leur quantité dépend de la forme galénique de la composition.

Comme huile, on peut citer les huiles minérales (vaseline), végétales (huile d'amande douce, de macadamia, de tournesol, de pépin de cassis), synthétiques comme les esters gras (palmitate d'octyle, lanolate d'isopropyle, les triglycérides dont ceux des acides caprique/caprylique), siliconées (cyclométhicone, polydiméthyl-siloxanes) ou fluorées.

Comme gélifiants et/ou agents de mise en suspension, on peut citer les argiles modifiées (bentones), les sels métalliques d'acides gras (stéarate d'aluminium), les polymères carboxyvinyliques, les polyglycéryl(méth)acrylates et les alcools gras (alcool cétylique).

Comme tensioactifs (émulsionnants et coémulsionnants), on peut utiliser les esters d'acide gras et de glycérol (stéarate de glycéryle) ou de sucre (stéarate de sorbitane).

Les compositions de l'invention peuvent aussi contenir divers ingrédients classiquement utilisés dans le domaine cosmétique, dermatologique ou dermopharmaceutique comme les pigments, les colorants, les cires (paraffine), les solvants, les conservateurs, les parfums, les actifs hydratants, les agents absorbant les rayons ultraviolets (filtres solaires, pigments), les charges.

Les différents constituants de la composition sont utilisés dans les quantités généralement utilisés dans les domaines considérés.

En particulier l'extrait de farine de maïs représente de 0,01 % à 10 % et mieux de 0,02 % à 5 % du poids total de la composition.

L'invention se rapporte aussi à l'utilisation cosmétique de la composition définie ci-dessus pour la régénération et/ou l'activation des cellules et/ou des tissus cutanés et donc pour la lutte contre et/ou prévention du vieillissement de la peau.

L'invention a encore pour objet un procédé cosmétique pour régénérer et/ou activer les cellules et/ou les tissus cutanés, consistant à appliquer sur la peau la composition définie précédemment.

Contrairement à toute attente, l'encapsulation d'extraits végétaux ne conduit pas forcément à une augmentation de l'efficacité de ces extraits sur la peau. Ainsi, le demandeur a pu constater, après des études longues et fastidieuses, que l'encapsulation d'extrait de farine de maïs augmentait de façon significative son efficacité contrairement à l'encapsulation d'autres extraits végétaux. Ceci ressort clairement des expériences mentionnées ci-après relatives à la prolifération de fibroblastes en présence ou non d'extraits végétaux encapsulés ou non. En outre, certains extraits encapsulés présentent même une cytotoxicité (c'est le cas du contre-exemple 1).

La prolifération cellulaire est évaluée par deux tests indirects reposant sur la coloration d'organites intracytoplasmiques uniquement dans les cellules vivantes :
- le Rouge Neutre, colorant rouge, incorporé au niveau des lysosomes,
- le MTT (3-(4,5-Diméthylthiazol-2-yl)-2,5-Diphényl tétrazolium bromide), substrat liquide jaune transformé en précipité bleu-violet par les enzymes mitochondriales.

Le rouge neutre est un colorant vital non toxique. Il traverse les membranes cellulaires et se fixe au niveau intracellulaire sur les groupements phosphates et/ou carboxyliques de la matrice lysosomiale. Seules les cellules vivantes retiennent le rouge neutre. La quantité de colorant retenue est donc proportionnelle à la quantité de lysosomes et sa mesure permet de donner une estimation du nombre de cellules vivantes.

La transformation du sel de tétrazolium MTT en cristaux bleus de formazan est réalisée par l'enzyme mitochondriale succinate-deshydrogénase. La quantité de formazan est donc proportionnelle à la quantité de mitochondries fonctionnelles et sa mesure permet de donner une estimation du nombre de cellules vivantes.

Après incorporation du colorant, les cellules sont lysées et l'intensité de la coloration est mesurée par spectrophotométrie. L'absorbance mesurée est alors proportionnelle au nombre d'organites fonctionnels et donc au nombre de cellules vivantes. Les résultats sont exprimés en pourcentage d'absorbance par rapport au témoin non traité. L'absorbance est mesurée à 550 nm.

Pour effectuer ces tests, on a utilisé des fibroblastes décongelés de peau humaine provenant d'une femme âgée de 27 ans isolés précédemment par la technique des explants.

L'ampoule à décongeler est extraite du container d'azote liquide et est aussitôt placée dans un bain-marie à 37°C. Dès que le contenu est redevenu liquide, il est transféré dans un tube à centrifuger contenant 40 ml de milieu de culture complet additionné de 10 % de sérum de veau foetal (SVF) (Gibco 013-06290M). Le tube est centrifugé 10 mn à 800 tr/mn, le surnageant est aspiré et le culot cellulaire est repris par une quantité optimale de milieu suivant le nombre de cellules congelées dans l'ampoule (8 ml pour un flacon de 25 cm³). Le flacon est placé à l'incubateur (Heraeus B 5061) à 37 °C sous 5 % de CO₂.

Le milieu de culture contenait du sérum de veau foetal : SVF (Gibco 013-06290M) à 10 % en poids dans un milieu de culture complet : milieu DMEM (Gibco 041-01965M) contenant 1 % de L-glutamine 200 mM (Gibco 043-05030D), 1 % de mélange antibiotique/antimycotique (Gibco 600-5240PG).

Lorsque les cellules se sont divisées et qu'elles recouvrent la totalité de la surface du flacon, elles doivent subir un repiquage encore appelé passage. Ainsi, le milieu est aspiré et les cellules sont lavées avec un tampon phosphate PBS sans calcium ni magnésium (Gibco 041-04190M). Le PBS est à son tour aspiré et remplacé par la solution de trypsine-éthylène diamine tétra-acétique (Gibco 043-05300H). Après environ 5 min d'incubation à 37°C, les cellules se détachent. Elles peuvent alors être remises dans du milieu complet à 10 % de SVF et être séparées dans deux nouveaux flacons qui sont alors remis à l'incubateur jusqu'au passage suivant.

Pour obtenir une bonne croissance cellulaire, le milieu de culture doit être renouvelé deux fois par semaine.

L'opération d'ensemencement des cellules pour les tests de croissance est la même que pour un repiquage mais dans ce cas les cellules sont ensemencées dans des plaques à 96 puits à raison de 20000 cellules/cm² soit 6400 cellules/puits ou encore 32000 cellules/ml. Le dénombrement des cellules est réalisé à l'aide d'une cellule de Malassez. Les plaques ensemencées sont placées 24 heures à l'incubateur.

Après 24 heures d'incubation, le milieu est éliminé et remplacé par les différentes solutions à tester et les différents témoins préparés dans du milieu 5 % SVF. Sur chaque plaque, chaque essai est réalisé en 6 exemplaires dans les 6 puits d'une même colonne afin de pouvoir donner une valeur moyenne. Quelque soit le nombre ou la nature des essais, la première colonne de chaque plaque ne contient pas de cellule et est affectée au blanc du test de coloration. La deuxième colonne est, quant à elle, réservée au "témoin non traité", c'est-à-dire au milieu 5 % SVF.

Les produits à tester sont encapsulés d'une part dans des vésicules de type "Lipides non ioniques" et d'autre part dans des vésicules de type "Liposome" suivant la méthode de Bangham décrite ci-après.

300 mg de lipides constitutifs de la membrane des vésicules sont pesés puis dissous dans 10 ml d'un mélange 50/50 de chloroforme et de méthanol. Après agitation, la solution est diluée 10 fois dans le mélange 50/50 chloroforme/méthanol, puis 1 ml de solution diluée est transvasé dans un ballon et additionné de 4 à 5 ml de solvant pour être évaporée à 40 °C. L'évaporation est poussée jusqu'au dépôt d'un film sec sur les parois du ballon.

3 ml de culture, contenant éventuellement un extrait végétal solubilisé, sont introduits dans le ballon mis sous agitation. Le ballon est secoué pendant une heure, la température étant maintenue à 40 °C. On laisse le milieu refroidir jusqu'à la température ambiante sous agitation.

On introduit alors une sonde à ultrasons dans la dispersion vésiculaire ainsi obtenue et on soumet celle-ci 10 min aux ultrasons. La suspension vésiculaire est ensuite filtrée sur un filtre 0,22 µm.

Pour les vésicules à base de lipides non-ioniques, on a utilisé 142,5 mg de cétyl éther de triglycéryle + 142,5 mg de cholestérol + 15 mg d'acylglutamate de Na, lipides notés ci-après Ni et pour les vésicules à base de lipides ioniques on a utilisé 300 mg de lécithine hydrogénée (Lipoid S75).

L'étude préliminaire des vésicules vides sur les fibroblastes a révélé une cytotoxicité des lipides aux fortes concentrations. La concentration en lipides de 2.10⁻⁵ moles dans le milieu de culture a été bien tolérée par les fibroblastes et a donc été retenue. Cette dose est constante pour tous les tests, aussi bien pour les lipides non-ioniques que pour les liposomes.

De nombreux produits végétaux ont été testés par le demandeur en présence des fibroblastes. Chaque produit a éte testé à deux concentrations différentes, à la fois dans les lipides non ioniques et dans les liposomes. Les concentrations sont celles indiquées précédemment sauf pour certaines substances qui étaient insolubles à la concentration choisie.

Chaque expérience contient un groupe "Témoin non traité", un groupe "vésicules vides", un groupe "produit libre", un groupe "produit libre associé aux vésicules vides". Ces différents groupes témoins sont comparés au produit encapsulé.

Les cellules sont cultivées en microplaques contenant 96 puits. Chaque essai est constitué par un groupe de 6 puits. Les essais ne sont donc pas indépendants les uns des autres. Le test statistique pour la comparaison simultanée de tous les groupes est le test d'analyse des variances à un facteur.

Les substances testées sur fibroblastes sont données dans le tableau 1 ci-après et les résultats de la croissance cellulaire sont donnés dans les tableaux 2 et 3 ci-après.

La légende des tableaux 2 et 3 est la suivante :
- P: = produit libre à concentration P
- [L]: = liposomes vides
- [L+P]: = P encapsulé dans des liposomes

Les valeurs spectrophotométriques sont exprimées en pourcentage par rapport au témoin non traité propre à chaque expérience (une expérience = une ligne de tableau).

Les tableaux présentent la totalité des résultats après 7 jours de contact entre les cellules et les produits.

Un résultat positif est une croissance cellulaire plus rapide avec le produit encapsulé comparé à la même concentration de produit libre. De plus, cette augmentation doit être statistiquement significative dans les deux tests au rouge neutre et au MTT.

**Tableau 1**

| **Noms des substances encapsulées** | | | |
|---|---|---|---|
| **Echantillons** | **Nom** | **Fournisseur** | **Composition chimique** |
| Contre-exemple 1 | Peptein VgW | Hormel | Hydrolysat de protéines de blé (PM = 2000) à 20 % dans l'eau |
| Contre-exemple 2 | Tofupro U | IKEDA Corporation | Hydrolysat de protéines de soja à 20 % dans l'eau |
| Exemple | Extrait Filatov "végétal lipophilisé" | Solabia | Extrait aqueux de farine de maïs deshuilé en poudre |

**Tableau 2**

| **Test des substances au Rouge Neutre** | | | |
|---|---|---|---|
| **Produit P** | **P** | **[L]** | **[L+P]** |
| Contre-exemple 1 0,5 % | 109 | | |
| | 119 | 128 | 0 |
| Contre-exemple 2 0,5 % | 214 | | |
| | 235 | 144 | 202 |
| Exemple 0,1 % | 152 | | |
| | 157 | 121 | 195 |

**Tableau 3**

| **Test des substances au MTT** | | | |
|---|---|---|---|
| **Produit P** | **P** | **[L]** | **[L+P]** |
| Contre-exemple 1 0,5 % | 94 | | |
| | 83 | 123 | 4 |
| Contre-exemple 2 0,5 % | 239 | | |
| | 274 | 168 | 202 |
| Exemple 0,1 % | 157 | | |
| | 158 | 118 | 194 |

Le fait que les fibroblastes se multiplient, in vitro, dans des quantités raisonnables en présence d'extrait de farine de maïs notamment aqueux encapsulé permet de dire que cet extrait sera bien supporté par les cellules et/ou tissus de la peau et qu'il aura une activité de régénération des cellules et/ou des tissus de la peau

Ainsi, l'extrait de farine de maïs, encapsulé va pouvoir avantageusement remplacer les extraits d'animaux (extraits placentaires notamment) jusqu'à ce jour utilisés dans les compositions destinées à lutter contre et/ou prévenir le vieillissement.

L'invention a aussi pour objet un procédé d'augmentation de l'efficacité d'extrait de farine de maïs sur la peau, consistant à encapsuler cet extrait dans des vésicules lipidiques de préférence à membrane formée de lipides ioniques, et à appliquer l'extrait encapsulé sur la peau.

On donne ci-après des exemples de formulation conformes à l'invention destinés à la régénération des tissus et/ des cellules cutanés. Dans ces exemples les vésicules sont obtenues comme décrites précédemment et introduites dans l'émulsion et/ou le gel à la fin de la fabrication, sous homogénéisation.

| **Exemple 1 : Fluide de soin pour le corps** | |
|---|---|
| - Extrait de farine de maïs | 0,5 g |
| - Lipides Ni | 10 g |
| - Huile de tournesol | 10 g |
| - Sel de sodium de l'acide pyrrolidone carboxylique | 2 g |
| - Conservateur | 0,3 g |
| - Polymère carboxyvinylique (Carbopol 940) | 0,4 g |
| - Triéthanolamine (neutralisant) | 0,4 g |
| - Parfum | 0,4 g |
| - Eau déminéralisée | qsp 100 g |

| **Exemple 2 : Fluide de soin solaire** | |
|---|---|
| - Lipides Ni | 8 g |
| - Extrait de farine de maïs | 0,5 g |
| - Huile d'amande douce | 22 g |
| - Paradiméthylaminobenzoate de 2-éthylhexyle (filtre) | 3 g |
| - Gomme adragante | 0,4 g |
| - Parfum | 0,4 g |
| - Eau déminéralisée | qsp 100 g |

| **Exemple 3 : Crème de soins pour le visage** | |
|---|---|
| - Cétyl éther de triglycéryle | 3;8 g |
| - β-sitostérol | 3,3 g |
| - Dicétylphosphate | 0,4 g |
| - Huile de jojoba | 25 g |
| - Parfum | 0,6 g |
| - Carbopol 940 | 0,2 g |
| - Triéthanolamine | 0,2 g |
| - Extrait de farine de maïs | 1,2 g |
| - Conservateur | 0,3 g |
| - Eau déminéralisée | qsp 100 g |

| **Exemple 4 : Crème de soin et/ou de traitement de la peau sèche** | |
|---|---|
| - Lécithine de soja | 12 g |
| - Cholestérol | 9 g |
| - Propylène glycol | 9 g |
| - Huile de silicone | 4 g |
| - Extrait de farine de maïs | 5 g |
| - Stabilisant | 0,30 g |
| - Parfum | 0,6 g |
| - Eau déminéralisée | qsp 100 g |

## Revendications

1. Composition cosmétique et/ou dermatologique contenant, dans un milieu cosmétiquement et/ou dermatologiquement acceptable, au moins un extrait de farine de maïs encapsulé dans des vésicules lipidiques.

2. Composition selon la revendication 1, caractérisée en ce que les vésicules lipidiques comportent une membrane formée de lipides ioniques.

3. Composition selon la revendication 1 ou 2, caractérisée en ce que l'extrait de farine représente de 0,01 % à 10 % du poids total de la composition.

4. Composition selon l'une des revendications précédentes, caractérisée en ce que l'extrait de farine représente de 0,02 % à 5 % du poids total de la composition.

5. Utilisation cosmétique de la composition conforme à l'une des revendications précédentes, pour la régénération et/ou l'activation des cellules et/ou des tissus cutanés.

6. Procédé cosmétique pour régénérer et/ou activer les cellules et/ou les tissus cutanés, consistant à appliquer sur la peau la composition conforme à l'une des revendications 1 à 4.

7. Procédé cosmétique pour lutter contre et/ou prévenir le vieillissement de la peau, consistant à appliquer sur la peau la composition conforme à l'une des revendications 1 à 4.

8. Procédé cosmétique d'augmentation de l'efficacité d'extrait de farine de maïs sur la peau, consistant à encapsuler cet extrait dans des vésicules lipidiques et à appliquer l'extrait encapsulé sur la peau.

9. Procédé selon la revendication 8, caractérisé en ce que les vésicules comportent une membrane formée de lipides ioniques.

## Claims

1. Cosmetic and/or dermatological composition containing, in a cosmetically and/or dermatologically acceptable medium, at least one corn flour extract encapsulated in lipid vesicles.

2. Composition according to Claim 1, characterized in that the lipid vesicles contain a membrane formed of ionic lipids.

3. Composition according to Claim 1 or 2, characterized in that the flour extract represents from 0.01 % to 10 % of the total weight of the composition.

4. Composition according to one of the preceding claims, characterized in that the flour extract represents from 0.02 % to 5 % of the total weight of the composition.

5. Cosmetic use of the composition in accordance with one of the preceding claims for the regeneration and/or the activation of skin cells and/or tissues.

6. Cosmetic process for regenerating and/or activating skin cells and/or tissues, which consists in applying to the skin the composition in accordance with one of Claims 1 to 4.

7. Cosmetic process for combating and/or preventing ageing of the skin, which consists in applying to the skin the composition in accordance with one of Claims 1 to 4.

8. Cosmetic process for enhancing the efficacy of corn flour extract on the skin, which consists in encapsulating this extract in lipid vesicles and in applying the encapsulated extract to the skin.

9. Process according to Claim 8, characterized in that the vesicles contain a membrane formed of ionic lipids.

## Patentansprüche

1. Kosmetische und/oder dermatologische Zusammensetzung, die in einem kosmetisch und/oder dermatologisch akzeptablen Medium mindestens einen in Lipidvesikeln verkapselten Maismehlextrakt enthält.

2. Zusammensetzung nach Anspruch 1, dadurch gekennzeichnet, daß die Lipidvesikeln eine aus ionischen Lipiden gebildete Membran aufweisen.

3. Zusammensetzung nach Anspruch 1 oder 2, dadurch gekennzeichnet, daß der Mehlextrakt 0,01 bis 10 % des Gesamtgewichts der Zusammensetzung ausmacht.

4. Zusammensetzung nach einem der vorhergehenden Ansprüche, dadurch gekennzeichnet, daß der Mehlextrakt 0,02 bis 5 % des Gesamtgewichts der Zusammensetzung ausmacht.

5. Kosmetische Verwendung der Zusammensetzung nach einem der vorhergehenden Ansprüche für die Regenerierung und/oder Aktivierung der Zellen und/oder der Gewebe der Haut.

6. Verfahren für die Regenerierung und/oder Aktivierung der Zellen und/oder Gewebe der Haut, das darin besteht, auf die Haut die Zusammensetzung nach einem der Ansprüche 1 bis 4 aufzutragen.

7. Kosmetisches Verfahren für die Bekämpfung und/oder Vermeidung der Alterung der Haut, das darin besteht, auf die Haut die Zusammensetzung nach einem der Ansprüche 1 bis 4 aufzutragen.

8. Kosmetisches Verfahren zur Erhöhung der Wirksamkeit eines Maismehlextrakts auf der Haut, das darin besteht, diesen Extrakt in Lipidvesikeln zu verkapseln und den verkapselten Extrakt auf die Haut aufzutragen.

9. Verfahren nach Anspruch 8, dadurch gekennzeichnet, daß die Vesikeln eine aus ionischen Lipiden gebildete Membran aufweisen.
